(19) Europäisches Patentamt
European Patent Office
Office européen des brevets

(11) **EP 3 327 673 B1**

(12) **EUROPÄISCHE PATENTSCHRIFT**

(45) Veröffentlichungstag und Bekanntmachung des Hinweises auf die Patenterteilung:
**06.03.2019 Patentblatt 2019/10**

(51) Int Cl.:
***G06T 11/00*** *(2006.01)*

(21) Anmeldenummer: **17197258.1**

(22) Anmeldetag: **19.10.2017**

(54) **ERZEUGEN VON HOCHAUFGELÖSTEN CT-BILDERN MIT SPEKTRALER INFORMATION**

GENERATION OF HIGH RESOLUTION CT IMAGES WITH SPECTRAL INFORMATION

GÉNÉRATION D'IMAGES CT À HAUTE RÉSOLUTION COMPORTANT DES INFORMATIONS SPECTRALES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.11.2016 DE 102016223698**

(43) Veröffentlichungstag der Anmeldung:
**30.05.2018 Patentblatt 2018/22**

(73) Patentinhaber: **Siemens Healthcare GmbH**
**91052 Erlangen (DE)**

(72) Erfinder:
• **Kappler, Steffen**
**91090 Effeltrich (DE)**
• **Allmendinger, Thomas**
**91301 Forchheim (DE)**

(56) Entgegenhaltungen:
**US-A1- 2013 251 220     US-A1- 2015 043 796**

• **PICHA SHUNHAVANICH ET AL: "Lossy Compression of Projection Dara from Photon Counting Detectors", 4TH INTERNATIONAL CONFERENCE ON IMAGE FORMATION IN X-RAY COMPUTED TOMOGRAPHY, 18. Juli 2016 (2016-07-18), Seiten 467-470, XP055461354, Bamberg, Germany**

**Beschreibung**

Die Erfindung betrifft ein Verfahren zum Erzeugen von CT-Bildern mit spektraler Information. Zudem betrifft die Erfindung ein Bilderzeugungssystem. Weiterhin betrifft die Erfindung ein Computertomographiesystem.

Mit Hilfe moderner bildgebender Verfahren werden häufig zwei-oder dreidimensionale Bilddaten erzeugt, die zur Visualisierung eines abgebildeten Untersuchungsobjekts und darüber hinaus auch für weitere Anwendungen genutzt werden können.

Häufig basieren die bildgebenden Verfahren auf der Erfassung von Röntgenstrahlung, wobei sogenannte Projektionsmessdaten erzeugt werden. Beispielsweise können Projektionsmessdaten mit Hilfe eines Computertomographie-Systems (CT-Systems) akquiriert werden. Bei CT-Systemen läuft gewöhnlich eine an einer Gantry angeordnete Kombination aus Röntgenquelle und gegenüberliegend angeordnetem Röntgendetektor um einen Messraum um, in dem sich das Untersuchungsobjekt (das im Folgenden ohne Beschränkung der Allgemeinheit als Patient bezeichnet wird) befindet. Das Drehzentrum (auch "Isozentrum" genannt) fällt dabei mit einer sogenannten Systemachse z zusammen. Bei einem oder mehreren Umläufen wird der Patient mit Röntgenstrahlung der Röntgenquelle durchstrahlt, wobei mit Hilfe des gegenüberliegenden Röntgendetektors Projektionsmessdaten bzw. Röntgenprojektionsdaten erfasst werden.

Die erzeugten Projektionsmessdaten sind insbesondere von der Bauart des Röntgendetektors abhängig. Röntgendetektoren wiesen gewöhnlich eine Mehrzahl an Detektionseinheiten auf, die meist in Form eines regelmäßigen Pixelarrays angeordnet sind. Die Detektionseinheiten erzeugen jeweils für auf die Detektionseinheiten auftreffende Röntgenstrahlung ein Detektionssignal, welches zu bestimmten Zeitpunkten hinsichtlich Intensität und spektraler Verteilung der Röntgenstrahlung analysiert wird, um Rückschlüsse auf das Untersuchungsobjekt zu erhalten und Projektionsmessdaten zu erzeugen.

Zur Detektion der Röntgenstrahlung können zum Beispiel sogenannte quantenzählende Detektoren verwendet werden. Bei quantenzählenden bzw. photonenzählenden Röntgendetektoren wird das Detektionssignal für Röntgenstrahlung hinsichtlich der Intensität und der spektralen Verteilung der Röntgenstrahlung in Form von Zählraten analysiert. Die Zählraten werden als Ausgabedaten eines sogenannten Detektorkanals zur Verfügung gestellt, der jeweils einer Detektionseinheit zugeordnet ist. Bei quanten- bzw. photonenzählenden Detektoren mit mehreren Energieschwellen erzeugt jeder Detektorkanal auf Basis des jeweiligen Detektionssignals der Detektionseinheit pro Projektion meist einen Satz von Zählraten. Der Satz von Zählraten kann dabei Zählraten für mehrere verschiedene, insbesondere gleichzeitig überprüfte Energieschwellwerte umfassen. Die Energieschwellwerte und die Anzahl der Energieschwellen, denen jeweils ein Energieschwellenwert zugeordnet ist, sind meist als Signalanalyseparameter zur Erfassung der Projektion vorgegeben.

Die Anwendung solcher photonenzählender Detektoren in der klinischen Computertomographie ermöglicht eine spektrale Bildgebung unter Verwendung polychromatischer Strahlungsquellen in typischerweise 2 bis 4 spektralen Bereichen. Diesen Bereichen entsprechen die bereits erwähnten zu überprüfenden Energieschwellwerte. Derartige Dual- oder Multi-Energie-CT-Verfahren ermöglichen die Identifikation und Quantifizierung unterschiedlicher Materialien, wie zum Beispiel Iod und Knochen, im Patienten.

Zudem kann mit photonenzählenden Detektoren eine deutlich erhöhte Detektorauflösung erreicht werden, welche etwa bei dem zwei- bis fünffachen der Auflösung herkömmlicher CT-Detektoren liegt.

Werden die beiden Vorteile eines quantenzählenden Detektors, also die spektrale Bildgebung und die höhere Auflösung, genutzt, so bringt das einen enormen Anstieg der zu verarbeitenden Datenmenge mit sich. Der Anstieg betrifft sowohl die Menge der bei einer Bildaufnahme entstehenden Rohdaten als auch die anschließende Verarbeitung der Rohdaten zu Bilddaten. Aufgrund der Übertragung der spektralen Informationen erhöht sich die Datenrate bei 2 bis 4 Kanälen um den Faktor 2 bis 4. Infolge der höheren Ortsauflösung um den Faktor 2 bis 5 erhöht sich die Datenrate um den Faktor $2^2$ bis $5^2$. Mithin kann sich die Datenmenge bei den genannten Bedingungen um einen Faktor von 8 bis 100 erhöhen. Hinzu kommt noch, dass die sogenannte Frame-Rate, d.h. die Frequenz, mit der einzelne Bildaufnahmen bei einer CT-Bildaufnahme aus verschiedenen Richtungen aufgenommen werden, an die höhere Ortsauflösung angepasst, d.h. erhöht werden muss, um die höhere Auflösung nutzen zu können, was die Datenmenge weiter erhöht.

Eine Möglichkeit, die Datenmengen bei der spektralen CT-Bildgebung zu begrenzen, besteht darin, die Detektorauflösung durch sogenanntes Pixel-Fusing, also das Zusammenfassen von mehreren Pixeln zu reduzieren. Alternativ kann auch die Anzahl der spektralen Bereiche eingeschränkt werden, um die zu übertragenden Datenmengen zu reduzieren. Eine weitere Möglichkeit der Beschränkung der Datenmengen besteht darin, die Flächennutzung eines Detektors zu reduzieren. Die genannten Maßnahmen erfordern jedoch vom Benutzer, vorab eine Entscheidung darüber zu treffen, welche Eigenschaften bei der Bildgebung besonders wichtig sind. Beispielsweise muss darüber entschieden werden, wie wichtig bei der Untersuchung eine Höchstauflösung oder die Verfügbarkeit spektraler Informationen oder die Größe des abzubildenden Bildbereichs sein sollen. Entsprechend müssen vor der Bildaufnahme Einstellungen vorgenommen werden, um die gewünschten Bildgebungsparameter zu erhalten. Dabei geht jedoch eine Optimierung einer der genannten Eigenschaften zu Lasten der anderen und umgekehrt.

Der Konferenzartikel Picha Shunhavanich and Norbert J.Pelc, "Lossy Compression of Projection Data from Photon Counting Detectors",4TH INTERNATIONAL CONFERENCE ON IMAGE FORMATION IN X-RAY COMPUTED TOMOGRAPHY, 18. Juli 2016, Seiten 467-470,Bamberg, Germany, offenbart ein Verfahren zur verlustbehafteten Datenkompression in einem photonenzählenden CT System. Das Verfahren verwendet eine prädiktive Kodierung, bei welcher ein Projektionswert eines bestimmten Energiefensters aus den umgebenden Detektorzellen des gleichen Energiefensters sowie Werten aus den übrigen Energiefenstern geschätzt wird. Das Residual zwischen geschätztem und gemessenen Projektionswert wird effizient kodiert an die Auswerteeinheit übertragen.

Es besteht daher das Problem, dass bei einer vollständigen Nutzung der Vorteile von photonenzählenden Detektoren, die zu übertragenden und zu verarbeitenden Datenmengen stark erhöht werden, so dass sich der Aufwand bei der Konzeption der notwendigen Hardware ebenfalls stark erhöht.

Diese Aufgabe wird durch ein Verfahren zum Erzeugen von CT-Bildern mit spektraler Information gemäß Patentanspruch 1, ein Bilderzeugungssystem gemäß Patentanspruch 11 und ein Computertomographiesystem gemäß Patentanspruch 12 gelöst.

Bei dem erfindungsgemäßen Verfahren zum Erzeugen von CT-Bilddaten mit spektraler Information von einem Untersuchungsbereich eines Patienten wird der Untersuchungsbereich mit polychromatischer Röntgenstrahlung bestrahlt. Daraufhin werden spektral aufgelöste Projektionsmessdaten von dem Untersuchungsbereich mit Hilfe eines photonenzählenden Detektors mit mehreren Energieschwellen erfasst. Die spektral aufgelösten Projektionsmessdaten umfassen einen ersten Projektionsmessdatensatz, welcher einer ersten Energieschwelle zugeordnet ist, und mindestens einen zweiten Projektionsmessdatensatz, welcher mindestens einer zweiten Energieschwelle zugeordnet ist. Auf Basis des mindestens einen zweiten Projektionsmessdatensatzes wird mindestens ein zweiter Projektionsmessdatensatz mit reduzierter Auflösung erzeugt. Dieser Vorgang erfolgt detektornah, d.h. in einer sich an den Detektor anschließenden, sich mit diesem mitbewegenden Datenreduktionseinheit. Anschließend werden der erste Projektionsmessdatensatz und der mindestens eine zweite Projektionsmessdatensatz mit reduzierter Auflösung an eine Bilderzeugungseinheit übermittelt. Die Bilderzeugungseinheit liegt außerhalb des sich mit der Drehbewegung der Gantry mitbewegenden Systems. Vorteilhaft kann bei dem erfindungsgemäßen Verfahren die Übertragung der Projektionsmessdatensätze zumindest teilweise mit reduzierter Auflösung und damit mit reduziertem Datenumfang geschehen. Nachfolgend wird in der Bilderzeugungseinheit die Auflösung des ersten Projektionsmessdatensatzes auf den mindestens einen zweiten Projektionsmessdatensatz mit reduzierter Auflösung und/oder auf einen auf dem mindestens einen zweiten Projektionsmessdatensatz mit reduzierter Auflösung basierenden Bilddatensatz übertragen. Mithin wird für einen auf dem ersten und dem mindestens einen zweiten Projektionsmessdatensatz basierenden spektralen Bilddatensatz eine dem ersten Projektionsmessdatensatz entsprechende Auflösung erreicht, so dass trotz der Reduktion der Datenmenge bei der Übertragung zwischen dem Detektor und der Bilderzeugungseinheit hoch aufgelöste spektrale Bilddaten erzielt werden.

Das erfindungsgemäße Bilderzeugungssystem umfasst einen quantenzählenden Detektor mit mehreren Energieschwellen zum Erfassen von spektral aufgelösten Projektionsmessdaten von einem Untersuchungsbereich eines Patienten. Die spektral aufgelösten Projektionsmessdaten umfassen einen ersten Projektionsmessdatensatz, welcher einer ersten Energieschwelle zugeordnet ist, und mindestens einen zweiten Projektionsmessdatensatz, welcher mindestens einer zweiten Energieschwelle zugeordnet ist. Teil des erfindungsgemäßen Bilderzeugungssystems ist auch eine Datenreduktionseinheit zum Erzeugen mindestens eines zweiten Projektionsmessdatensatzes mit reduzierter Auflösung auf Basis des mindestens einen zweiten Projektionsmessdatensatzes. Wie bereits erwähnt, ist die Datenreduktionseinheit detektornah angeordnet. Zudem umfasst das erfindungsgemäße Bilderzeugungssystem eine Datenübermittlungseinheit zum Übermitteln der Projektionsmessdaten von dem quantenzählenden Detektor an eine Bilderzeugungseinheit. Eine solche Datenübermittlungseinheit kann zum Beispiel eine Datenleitung, einen Schleifring zur Übertragung der Daten zwischen dem Detektor und dem stationären Teil des Bilderzeugungssystems oder ein Funkübertragungssystem umfassen. Das erfindungsgemäße Bilderzeugungssystem umfasst weiterhin eine Bilderzeugungseinheit zum Übertragen der Auflösung des ersten Projektionsmessdatensatzes auf den mindestens einen zweiten Projektionsmessdatensatz mit reduzierter Auflösung und/oder auf einen auf dem mindestens einen zweiten Projektionsmessdatensatz mit reduzierter Auflösung basierenden Bilddatensatz. Ferner ist die Bilderzeugungseinheit dazu eingerichtet, Bilddaten auf Basis der übermittelten Projektionsmessdaten zu rekonstruieren. Die Übertragung der Auflösung kann vor der Rekonstruktion der Bilddaten erfolgen, sie kann aber auch zwischen einzelnen Schritten der Bildrekonstruktion erfolgen.

Das erfindungsgemäße Computertomographiesystem weist eine Röntgenquelle zum Bestrahlen eines Untersuchungsbereichs eines Patienten mit polychromatischer Röntgenstrahlung und ein erfindungsgemäßes Bilderzeugungssystem auf.

Einige wesentliche Komponenten des erfindungsgemäßen Bilderzeugungssystems können in Form von Softwarekomponenten ausgebildet sein. Dies betrifft insbesondere die Datenreduktionseinheit und die Bilderzeugungseinheit.

Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein.

Eine weitgehend softwaremäßige Realisierung hat den Vorteil, dass auch schon bisher verwendete Computertomographiesysteme auf einfache Weise durch ein Software-Update nachgerüstet werden können, um auf die erfindungsgemäße Weise zu arbeiten. Insofern wird die Aufgabe auch durch ein Computerprogrammprodukt gelöst, welches direkt in einen Speicher eines Computertomographiesystems ladbar ist, mit Programmcodeabschnitten, um alle Schritte des erfindungsgemäßen Verfahrens auszuführen, wenn das Programm in dem Computertomographiesystem ausgeführt wird.

Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zu dem Computertomographiesystem und/oder zur Speicherung an oder in dem Computertomographiesystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen. Auch eine drahtlose Übertragung des Computerprogramms ist möglich.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren zugehörigen Beschreibungsteilen weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

In einer Ausgestaltung des erfindungsgemäßen Verfahrens zum Erzeugen von CT-Bilddaten mit spektraler Information von einem Untersuchungsbereich eines Patienten wird ein Kombinationsbild erzeugt. Hierzu werden ein erster Bilddatensatz und mindestens ein zweiter Bilddatensatz auf Basis des ersten Projektionsmessdatensatzes und auf Basis des mindestens einen zweiten Projektionsmessdatensatzes mit reduzierter Auflösung rekonstruiert. Dieser Vorgang erfolgt unter Anwendung der Übertragung der Auflösung des ersten Projektionsmessdatensatzes auf den mindestens einen zweiten Projektionsmessdatensatz mit reduzierter Auflösung und/oder auf den mindestens einen zweiten Bilddatensatz. Nachfolgend wird ein Kombinationsbild durch Kombinieren des ersten Bilddatensatzes und des mindestens einen zweiten Bilddatensatzes erzeugt. Das Kombinationsbild kann zum Beispiel durch Differenzbildung oder gewichtete Addition einzelner Bilddatensätze, d.h. insbesondere des ersten und des mindestens einen zweiten Bilddatensatzes, erzeugt werden. Ein solches Kombinationsbild kann zum Beispiel zur kontrastverstärkten Darstellung einzelner Materialarten, wie zum Beispiel Iod oder Knochenmaterial, genutzt werden. Vorteilhaft kann dieses Kombinationsbild bei dem erfindungsgemäßen Verfahren trotz Reduktion der zu übertragenden Datenmenge mit hoher Auflösung erzeugt werden.

In einer bevorzugten Ausgestaltung des erfindungsgemäßen Verfahrens zum Erzeugen von CT-Bilddaten mit spektraler Information von einem Untersuchungsbereich eines Patienten weist die erste Energieschwelle einen niedrigeren Energiewert aufweist als die mindestens eine zweite Energieschwelle. Bei dieser Ausgestaltung umfasst der erste Projektionsmessdatensatz die größte Menge an struktureller Information und wird deshalb mit unveränderter Auflösung übertragen. Dagegen werden Projektionsmessdaten, welche der mindestens einen zweiten Energieschwelle zugeordnet sind, mit reduzierter Auflösung übertragen und erhalten im Nachhinein strukturelle Informationen von dem ersten Projektionsmessdatensatz. Vorteilhaft wird bei dieser Vorgehensweise eine optimale Auflösung struktureller Details kombiniert mit einer Reduktion der zu übertragenden Datenmenge erreicht.

In einer Variante des erfindungsgemäßen Verfahrens zum Erzeugen von CT-Bilddaten mit spektraler Information von einem Untersuchungsbereich eines Patienten wird die reduzierte Auflösung des mindestens einen zweiten Projektionsmessdatensatzes durch Reduktion der Auflösung in mindestens einer der folgenden Richtungen des Detektors erzeugt wird:

- in Kanalrichtung,
- in Zeilenrichtung,
- in Projektionsrichtung.

Die Zeilenrichtung eines Detektors verläuft üblicherweise in z-Richtung, d.h. in Richtung der Systemachse eines CT-Systems. Die Kanalrichtung verläuft orthogonal zu der Zeilenrichtung und tangential zur Detektorfläche. Dagegen verläuft die Projektionsrichtung orthogonal zur Detektorfläche und damit auch zur orthogonal zur Zeilenrichtung und zur Kanalrichtung.

Eine besonders stark reduzierte Auflösung des mindestens einen zweiten Projektionsmessdatensatzes kann durch Reduktion der Auflösung in Kanalrichtung, Zeilenrichtung und Projektionsrichtung zugleich erfolgen.

Im Rahmen des erfindungsgemäßen Verfahrens zum Erzeugen von CT-Bilddaten mit spektraler Information von einem Untersuchungsbereich eines Patienten kann zusätzlich der erste Projektionsmessdatensatz mit einer kürzeren Frame-Zeit akquiriert werden als der mindestens eine zweite Projektionsmessdatensatz. Auf diese Weise wird der höheren Auflösung, mit der der erste Projektionsmessdatensatz übertragen wird, Rechnung getragen. Da es aufgrund der Bewegung des Detektors bei der Akquisition der Projektionsmessdaten in Abhängigkeit von der Frame-Zeit zu einem Schärfeverlust ("Verschmierung") kommt, kann die erhöhte Auflösung des photonenzählenden Detektors nur dann effizient genutzt werden, wenn die Frame-Zeit, also die Zeit, in der eine Bildaufnahme von dem Untersuchungsbereich aus einer Richtung erfolgt, entsprechend verkürzt wird. Vorteilhaft wird also bei dieser Variante eine qualitativ besonders gute Bildaufnahme erzielt.

In einer Ausgestaltung des erfindungsgemäßen Verfahrens zum Erzeugen von CT-Bilddaten mit spektraler Information von einem Untersuchungsbereich eines Patienten kann der Transfer der höheren Auflösung mit Hilfe eines Bandfilters erfolgen. Hierbei kann eine Regularisierung des Transfers durch Gradientenvergleich sinnvoll sein. Mit Hilfe des Gradiententerms kann nachträglich ein Unterschied der Härte der Röntgenstrahlen bei unterschiedlichen Energieschwellen und der damit verbundenen Kontraste, der bei der Übertragung der Auflösung von der ersten Energieschwelle auf höhere Energieschwellen verloren geht, zurückgewonnen werden.

Der Transfer der höheren Auflösung kann im Rahmen der Erzeugung der Bilddaten beispielsweise in einem der folgenden Prozessstadien erfolgen:

- vor oder nach Vorverarbeitung der Projektionsmessdaten,
- nach einer projektions-basierten Materialzerlegung oder einer anderen spektralen Anwendung,
- vor oder nach Rückprojektion der Projektionsmessdaten,
- nach einer bild-basierten Materialzerlegung oder einer anderen spektralen Anwendung
- während eines der Schritte einer Materialzerlegung oder einer anderen spektralen Anwendung mittels iterativer Rekonstruktion.

Vorteilhaft kann der Auflösungstransfer also je nach Bedarf in unterschiedlichen Prozessstadien vorgenommen werden.

In einer bevorzugten Ausgestaltung des erfindungsgemäßen Verfahrens zum Erzeugen von CT-Bilddaten mit spektraler Information von einem Untersuchungsbereich eines Patienten werden zum Übertragen der Auflösung des ersten Projektionsmessdatensatzes auf den mindestens einen zweiten Projektionsmessdatensatz mit reduzierter Auflösung und/oder auf einen auf dem mindestens einen zweiten Projektionsmessdatensatz mit reduzierter Auflösung basierenden Bilddatensatz die folgende Vorgehensweise angewandt: Zunächst wird ein erster Projektionsmessdatensatz mit reduzierter Auflösung auf Basis des ersten Projektionsmessdatensatzes erzeugt. Dann werden höher aufgelöste Differenzdaten auf Basis einer Subtraktion des ersten Projektionsmessdatensatzes und des ersten Projektionsmessdatensatzes mit reduzierter Auflösung erzeugt. Schließlich werden die höher aufgelösten Differenzdaten, eventuell mit einem Normierungsfaktor, umfassend einen Quotienten aus dem zweiten Projektionsmessdatensatz mit reduzierter Auflösung und dem ersten Projektionsmessdatensatz mit reduzierter Auflösung multipliziert, zu dem zweiten Projektionsmessdatensatz mit reduzierter Auflösung addiert. Dabei entsteht dann wieder ein zweiter Projektionsmessdatensatz mit erhöhter Auflösung. Der mit dem ersten Projektionsmessdatensatz zur Erzeugung eines hoch aufgelösten, spektrale Information umfassenden Bildes kombiniert werden kann.

Die Erfindung wird im Folgenden unter Hinweis auf die beigefügten Figuren anhand von Ausführungsbeispielen noch einmal näher erläutert. Es zeigen:

FIG 1 ein Flussdiagramm, welches ein Verfahren zum Erzeugen von CT-Bilddaten mit spektraler Information von einem Untersuchungsbereich eines Patienten gemäß einem Ausführungsbeispiel der Erfindung veranschaulicht,

FIG 2 eine schematische Darstellung von erfassten Projektionsmessdaten und in ihrer Auflösung reduzierten Projektionsmessdaten,

FIG 3 eine schematische Darstellung einer Übertragung der Auflösung von höher aufgelösten Projektionsmessdaten auf niedriger aufgelöste Projektionsmessdaten,

FIG 4 eine schematische Darstellung eines Bilderzeugungssystems gemäß einem Ausführungsbeispiel der Erfindung,

FIG 5 eine schematische Darstellung eines Computertomographiesystems gemäß einem Ausführungsbeispiel der Erfindung.

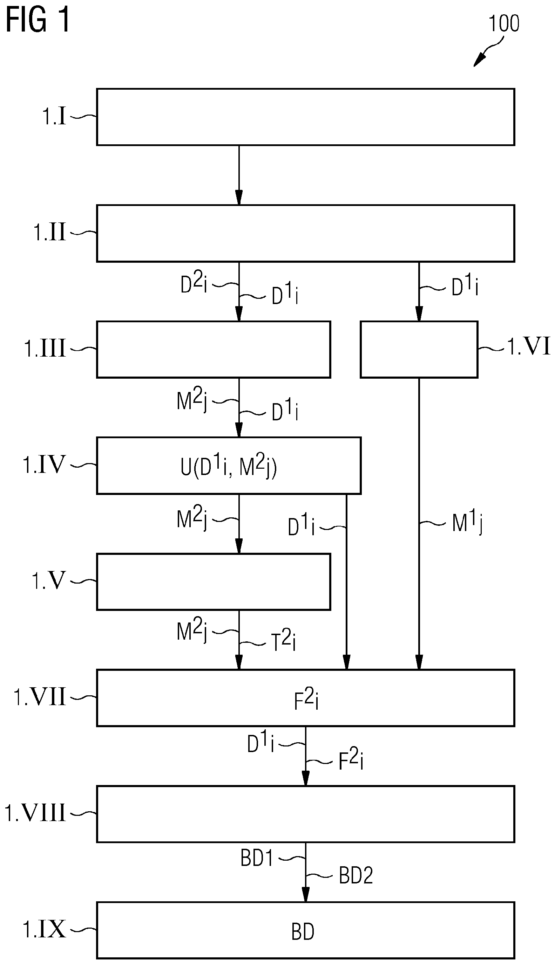

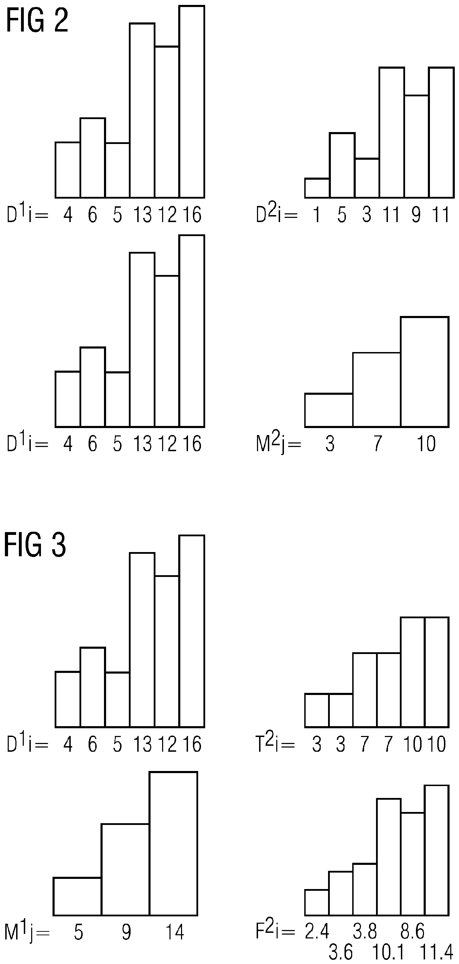

In FIG 1 ist ein Flussdiagramm 100 gezeigt, welches einzelne Schritte eines Verfahrens zum Erzeugen von CT-Bilddaten mit spektraler Information von einem Untersuchungsbereich eines Patienten gemäß einem Ausführungsbeispiel der Erfindung verdeutlicht. Einzelne Schritte des in FIG 1 gezeigten Verfahrens sind in FIG 2 und FIG 3 mit Hilfe von Balkendiagrammen graphisch veranschaulicht. Bei dem Schritt 1.I wird zunächst im Rahmen einer CT-Bildaufnahme ein Untersuchungsbereich mit Röntgenstrahlung beaufschlagt. Bei dem Schritt 1.II werden darauf spektral aufgelöste Projektionsmessdaten $D^1_i$, $D^2_i$ von dem Untersuchungsbereich mit Hilfe eines photonenzählenden Detektors mit mehreren Energieschwellen erfasst. Bei diesem Vorgang werden erste und zweite Projektionsmessdaten $D^1_i$, $D^2_i$ erfasst, die unterschiedlichen Energieschwellen des Röntgendetektors zugeordnet sind. Der Index i steht dabei für unterschiedliche Kanäle, welchen unterschiedliche Pixel oder Subpixel zugeordnet sind und über die ein von dem Detektor zu erfassender Bildbereich simultan erfasst wird. Der hochgestellte Index 1 bzw. 2 steht dagegen für die Zuordnung der ersten Projektionsmessdaten $D^1_i$ zur ersten Energieschwelle und der zweiten Projektionsmessdaten $D^2_i$ zur zweiten Energieschwelle.

In FIG 2 ist dieser Vorgang der Anschaulichkeit halber für 6 Kanäle, also i = 0 bis 5 und zwei Energieschwellen gezeigt. Jedem der Kanäle ist jeweils ein Balken eines Balkendiagramms zugeordnet. Oben links in FIG 2 sind die ersten Projektionsmessdaten $D^1_i$ für die erste Schwelle dargestellt. Hierzu sind den einzelnen Kanälen unterschiedliche Zählraten zugeordnet, die unterschiedlichen Abschwächungen der erfassten Röntgenstrahlen an unterschiedlichen Orten entsprechen. Beispielsweise weist ein erster Kanal eine Zählrate $D^1_0 = 4$ auf, ein zweiter Kanal eine Zählrate $D^1_0 = 6$ auf usw..

Oben rechts in FIG 2 sind die Zählraten für unterschiedliche Kanäle für die zweiten Projektionsmessdaten $D^2_i$ für die zweite Energieschwelle dargestellt. Hierzu sind den einzelnen Kanälen unterschiedliche Zählraten zugeordnet, die unterschiedlichen Abschwächungen der erfassten Röntgenstrahlen an unterschiedlichen Orten entsprechen. Beispielsweise weist ein erster Kanal eine Zählrate $D^2_0 = 1$ auf, ein zweiter Kanal eine Zählrate $D^1_0 = 5$ auf usw.. Gut zu erkennen ist, dass die Zählraten für die einzelnen Kanäle der zweiten Projektionsmessdaten $D^2_0$ niedriger sind als die Zählraten der entsprechenden Kanäle der ersten Projektionsmessdaten $D^1_0$. Dies liegt daran, dass die erste Energieschwelle niedriger liegt als die zweite Energieschwelle, so dass ein Teil der bei der ersten Energieschwelle noch detektierten Röntgenquanten unterhalb der zweiten Energieschwelle liegen und bei der Akquisition der zweiten Projektionsmessdaten nicht mehr erfasst werden.

Bei dem in FIG 1 veranschaulichten Verfahren werden nun bei dem Schritt 1.III die mit der niedrigsten Energieschwelle erfassten ersten Projektionsmessdaten $D^1_i$ in ihrer vollen Auflösung belassen, während die mit der höheren Energieschwelle erfassten zweiten Projektionsmessdaten $D^2_i$ in eine Hilfsgröße, im Folgenden als zweite schwächer aufgelöste Projektionsmessdaten $M^2_j$ bezeichnet, umgewandelt werden. Dieser Vorgang ist in FIG 2 mit den beiden unteren Schaubildern veranschaulicht. Unten links in FIG 2 sind nochmals die bei dem Schritt 1.II erfassten ersten Projektionsmessdaten $D^1_i$ für sechs Kanäle gezeigt, welche sich bei dem Schritt 1.III nicht verändert haben. Unten rechts in FIG 2 sind zum Vergleich die schwächer aufgelösten Projektionsmessdaten $M^2_3$ für j = 0 bis 2 gezeigt, welche auf Basis der zweiten Projektionsmessdaten $D^2_i$ erzeugt wurden. Die erzeugten schwächer aufgelösten Projektionsmessdaten $M^2_j$ sind dabei jeweils über zwei Kanäle gemittelt, d.h.

$$M^2_j = \frac{D^2_{2j} + D^2_{2j+1}}{2}, \quad j \, \varepsilon \, \{0...N/2-1\}.$$

Dabei ist N eine ganze Zahl, in dem in FIG 2 gezeigten Beispiel ist N = 5.

Die vorgenannten Schritte erfolgen alle detektornah, d.h. entweder im Detektor oder in einer sich mit dem Detektor mitdrehenden Recheneinheit. Bei dem Schritt 1.IV erfolgt nun eine Übermittlung $U(D^1_i, M^2_j)$ sowohl der höher aufgelösten ersten Projektionsmessdaten $D^1_i$ als auch der schwächer aufgelösten Projektionsmessdaten $M^2_j$ an eine von dem Detektor getrennte, vorzugsweise in einer Steuerungseinrichtung des betreffenden CT-Systems festinstallierte Bilderzeugungseinheit. Da die Auflösung der zweiten Projektionsmessdaten $D^2_i$ reduziert wurde, kann die Datenübertragungsrate bei der Übermittlung der Projektionsmessdaten $D^1_i$, $M^2_j$ nun kleiner ausfallen.

Bei dem Schritt 1.V wird anschließend auf Basis der schwächer aufgelösten Projektionsmessdaten $M^2_j$ eine Hilfsgröße $T^2_i$ ermittelt, welche dieselbe Kanalzahl aufweist wie die höher aufgelösten ersten Projektionsmessdaten $D^1_i$. Dabei ergibt sich die Hilfsgröße $T^2_i$ wie folgt:

$$T^2_i = M^2_{floor(i/2)}, \quad i \, \varepsilon \, \{0...N-1\}.$$

Dabei ist in dem in FIG 3 gezeigten Beispiel N = 5 und "floor" ist eine Abrundungsfunktion, welche eine Zahl auf die nächstkleinere ganze Zahl abrundet. Wie in FIG 3 in der Teilzeichnung oben rechts zu erkennen ist, wurden zur

Erzeugung der Hilfsgröße $T^2_i$ die Werte der schwächer aufgelösten Projektionsmessdaten $M^2_j$ einfach beibehalten und die Anzahl der Kanäle verdoppelt. Dabei wurde jedem der drei Kanäle der schwächer aufgelösten Projektionsmessdaten $M^2_j$ einfach ein zusätzlicher Kanal hinzugefügt, der denselben Zählratenwert erhalten hat wie der bereits vorhandene Kanal.

Weiterhin wird bei dem Schritt 1.VI eine weitere Hilfsgröße, im Folgenden als schwächer aufgelöste erste Projektionsmessdaten $M^1_j$ bezeichnet, auf Basis der ersten Projektionsmessdaten $D^1_i$ berechnet, wobei die Auflösung der Hilfsgröße $M^1_j$ im Vergleich zu der Auflösung der ersten Projektionsmessdaten $D^1_i$ halbiert wird. Die ersten schwächer aufgelösten Projektionsmessdaten $M^1_j$ ergeben sich wie folgt:

$$M^1_j = \frac{D^1_{2j} + D^1_{2j+1}}{2}, \quad j\, \varepsilon\, \{0...N/2-1\}.$$

Die schwächer aufgelösten ersten Projektionsmessdaten $M^1_j$ sind in FIG 3 unten links veranschaulicht. Wie dort zu erkennen ist, sind die Zählraten der Kanäle 0, 1, 2 einfach Durchschnittswerte benachbarter Kanäle der höher aufgelösten Projektionsmessdaten $D^1_i$.

Anschließend werden bei dem Schritt 1.VII um die Strukturinformation der ersten Projektionsmessdaten $D^1_i$ bereicherte korrigierte hochaufgelöste zweite Projektionsmessdaten $F^2_i$ erzeugt. Die korrigierten hochaufgelösten zweiten Projektionsmessdaten $F^2_i$ ergeben sich wie folgt:

$$F^2_i = \frac{M^2_{\text{floor}(i/2)}}{M^1_{\text{floor}(i/2)}}\left(D^1_i - M^1_{\text{floor}(i/2)}\right) + T^2_i, \quad i\, \varepsilon\, \{0...N-1\}.$$

Die Übertragung der Strukturinformation der hochaufgelösten ersten Projektionsmessdaten $D^1_i$ erfolgt durch Hinzufügen der Differenz zwischen den ersten hochaufgelösten Projektionsmessdaten $D^1_i$ und den niedrig aufgelösten ersten Projektionsmessdaten $M^1_{\text{floor}(i/2)}$, wobei diese Differenz noch mit dem Quotienten aus den beiden Hilfsgrößen $M^2_{\text{floor}(i/2)}$ und $M^1_{\text{floor}(i/2)}$ normiert wird.

Bei dem Schritt 1.VIII werden dann auf Basis der hochaufgelösten ersten Projektionsmessdaten $D^1_i$ und der korrigierten hochaufgelösten zweiten Projektionsmessdaten $F^2_i$ unterschiedlichen Spektren zugeordnete Bilddatensätze BD1, BD2 rekonstruiert.

Schließlich wird bei dem Schritt 1.IX ein Differenzbild BD auf Basis der zugeordneten Bilddatensätze BD1, BD2 erzeugt.

Wie in FIG 3 unten rechts zu erkennen ist, weisen die um die Strukturinformation der ersten Projektionsmessdaten $D^1_i$ bereicherten, korrigierten hochaufgelösten zweiten Projektionsmessdaten $F^2_i$ ähnlich wie die ursprünglichen zweiten Projektionsmessdaten $D^2_i$ eine Kante zwischen dem Kanal 2 und dem Kanal 3 auf, wobei der Zählratenwert zwischen Kanal 2 und Kanal 3 von 3.8 auf 10.1 ansteigt. Zum Vergleich steigt der Zählratenwert bei den ursprünglichen zweiten Projektionsmessdaten $D^2_i$ von Kanal 2 (dritter Kanal) zu Kanal 3 (vierter Kanal) von 3 auf 11 an. Der starke Werteanstieg kann zum Beispiel eine Kante repräsentieren, welche ansonsten bei der Übertragung der niedrig aufgelösten zweiten Projektionsmessdaten $M^2_j$ zwischen einem rotierenden Detektor und einer ruhenden Auswertungseinheit, welche zum Beispiel eine Bildrekonstruktionsfunktion wahrnimmt, verloren gegangen wäre. Auf diese Weise werden feinere Strukturinformationen in den zweiten Projektionsmessdaten im Nachhinein rekonstruiert, ohne dass bei der Datenübertragung eine erhöhte Datenmenge übertragen werden muss.

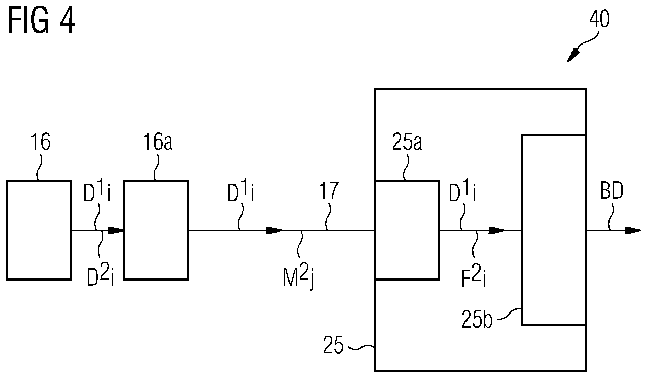

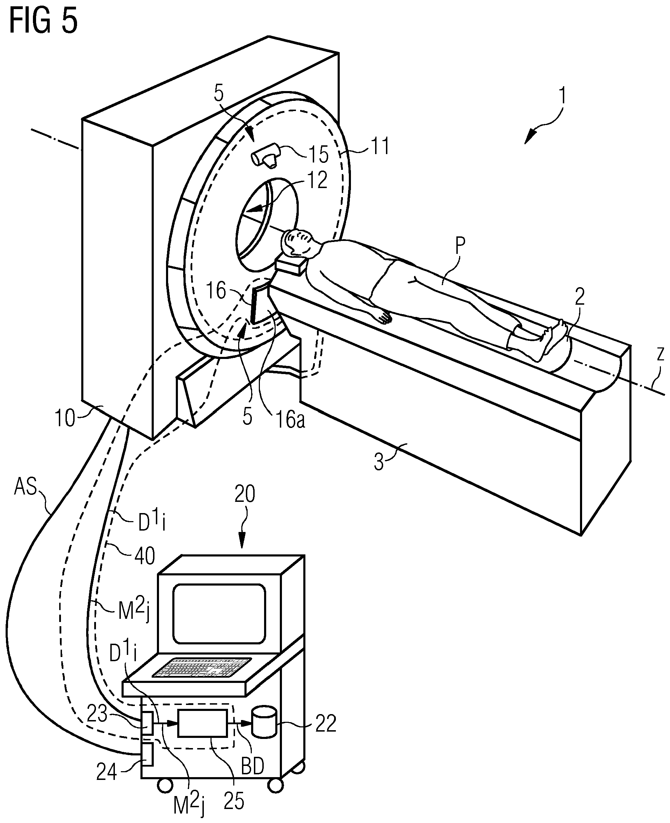

In FIG 4 ist ein Bilderzeugungssystem 40 gemäß einem Ausführungsbeispiel der Erfindung gezeigt. Das Bilderzeugungssystem 40 umfasst einen quantenzählenden Detektor 16. Der quantenzählende Detektor 16 weist mehrere Energieschwellen auf und erfasst spektral aufgelöste erste und zweite Projektionsmessdaten $D^1_i$, $D^2_i$ von einem Untersuchungsbereich eines Patienten. An den Detektor schließt sich eine Datenreduktionseinheit 16a an, welche dazu dient einen Projektionsmessdatensatz $M^2_j$ mit reduzierter Auflösung auf Basis eines erfassten Projektionsmessdatensatzes $D^2_i$, welcher einer höheren Energieschwelle zugeordnet ist, zu erzeugen. Der oder die Projektionsmessdatensätze $M^2_j$ mit reduzierter Auflösung sowie mindestens ein in seiner Auflösung nicht reduzierter Projektionsmessdatensatz $D^1_i$ werden mit Hilfe einer Datenübermittlungseinheit 17 von der Datenreduktionseinheit 16a an eine Bilderzeugungseinheit 25 übermittelt. Die Datenübermittlungseinheit kann zum Beispiel eine Übertragungsleitung und einen Schleifring zur Übertragung der Projektionsmessdaten $D^1_i$, $M^2_j$ von der mitrotierenden Datenreduktionseinheit 16a an die ruhende Bilderzeugungseinheit 25 aufweisen. Die Bilderzeugungseinheit 25 kann zum Beispiel Teil einer Steuerungseinrichtung eines CT-Systems sein (siehe FIG 5) und sie weist eine Übertragungseinheit 25a auf, die dazu dient, die Auflösung des in seiner Auflösung nicht reduzierten ersten Projektionsmessdatensatzes $D^1_i$ auf den zweiten Projektionsmessdatensatz

$M^2_j$ mit reduzierter Auflösung zu übertragen. Dabei werden korrigierte zweite Projektionsmessdaten $F^2_i$ mit erhöhter Auflösung erzeugt. Die Projektionsmessdaten $D^1_i$, $F^2_i$ mit hoher Auflösung werden dann an eine Bildrekonstruktionseinheit 25b übermittelt, welche dazu dient, Bilddaten BD auf Basis der Projektionsmessdaten $D^1_i$, $F^2_i$ zu rekonstruieren.

In FIG 5 ist ein Computertomographiesystem 1 gezeigt, welches das in FIG 4 gezeigte Bilderzeugungssystem 40 (in FIG 5 mit gestrichelten Linien markiert) umfasst. Das CT-System 1 besteht dabei im Wesentlichen aus einer üblichen Scaneinheit 10, in welcher an einer Gantry 11 eine Projektionsdatenakquisitionseinheit 5 mit einem spektral auflösenden Detektor 16 und einer dem Detektor 16 gegenüberliegenden Röntgenquelle 15, welche polychromatische Röntgenstrahlen emittiert, um einen Messraum 12 umläuft. Vor der Scaneinheit 10 befindet sich eine Patientenlagerungseinrichtung 3 bzw. ein Patiententisch 3, dessen oberer Teil 2 mit einem darauf befindlichen Patienten P zur Scaneinheit 10 verschoben werden kann, um den Patienten P durch den Messraum 12 hindurch relativ zum Detektorsystem 16 zu bewegen. Angesteuert werden die Scaneinheit 10 und der Patiententisch 3 durch eine Steuereinrichtung 20, von der aus über eine übliche Steuerschnittstelle 24 Akquisitionssteuersignale AS kommen, um das gesamte System gemäß vorgegebener Messprotokolle in der herkömmlichen Weise anzusteuern. Im Fall einer Spiralakquisition ergibt sich durch eine Bewegung des Patienten P entlang der z-Richtung, welche der Systemachse z längs durch den Messraum 12 entspricht, und den gleichzeitigen Umlauf der Röntgenquelle 15 für die Röntgenquelle 15 relativ zum Patienten P während der Messung eine Helixbahn. Parallel läuft dabei immer gegenüber der Röntgenquelle 15 der Detektor 16 mit, um spektral aufgelöste Projektionsmessdaten $D^1_i$, $D^2_i$ zu erfassen, die dann zur Rekonstruktion von Volumen- und/oder Schicht-Bilddaten genutzt werden. Ebenso kann auch ein sequentielles Messverfahren durchgeführt werden, bei dem eine feste Position in z-Richtung angefahren wird und dann während eines Umlaufs, eines Teilumlaufs oder mehrerer Umläufe an der betreffenden z-Position die erforderlichen spektral aufgelösten Projektionsmessdaten $D^1_i$, $D^2_i$ erfasst werden, um ein Schnittbild an dieser z-Position zu rekonstruieren oder um aus den Projektionsmessdaten $D^1_i$, $D^2_i$ mehrerer z-Positionen Bilddaten zu rekonstruieren. Das erfindungsgemäße Verfahren ist grundsätzlich auch an anderen CT-Systemen, z.B. mit mehreren Röntgenquellen und/oder Detektoren und/oder mit einem einen vollständigen Ring bildenden Detektor, einsetzbar. Beispielsweise lässt sich das erfindungsgemäße Verfahren auch auf ein System mit unbewegtem Patiententisch und in z-Richtung bewegter Gantry (einer sogenannten Sliding Gantry) anwenden.

Die vom Detektor 16 akquirierten Projektionsmessdaten $D^1_i$, $D^2_i$ (im Folgenden auch Rohdaten genannt) werden von einer detektorseitig angeordneten Datenreduktionseinheit 16a, wie oben beschrieben, reduziert und es werden Projektionsmessdaten $D^1_i$, $M^2_j$ über eine Datenschnittstelle 23 an die Steuereinrichtung 20 übergeben. Diese Projektionsmessdaten $D^1_i$, $M^2_j$ werden dann, gegebenenfalls nach einer geeigneten Vorverarbeitung (z. B. Filterung und/oder Strahlaufhärtungskorrektur), in einer Bilderzeugungseinheit 25 weiterverarbeitet, die in diesem Ausführungsbeispiel in der Steuereinrichtung 20 in Form von Software auf einem Prozessor realisiert ist. Diese Bilderzeugungseinheit 25 erhöht die Auflösung der in ihrer Auflösung reduzierten zweiten Projektionsmessdaten $M^2_j$ wieder auf die in FIG 1 veranschaulichte Art und Weise und rekonstruiert auf Basis der hoch aufgelösten Projektionsmessdaten mit Hilfe eines Rekonstruktionsverfahrens Bilddaten BD. Als Rekonstruktionsverfahren kann zum Beispiel ein auf der gefilterten Rückprojektion basierendes Rekonstruktionsverfahren verwendet werden.

Die erzeugten Bilddaten werden anschließend in einem Speicher 22 der Steuereinrichtung 20 hinterlegt und/oder in üblicher Weise auf dem Bildschirm der Steuereinrichtung 20 ausgegeben. Sie können auch über eine in FIG 5 nicht dargestellte Schnittstelle in ein an das Computertomographiesystem 1 angeschlossenes Netz, beispielsweise ein radiologisches Informationssystem (RIS), eingespeist und in einem dort zugänglichen Massenspeicher hinterlegt oder auf dort angeschlossenen Druckern oder Filming-Stationen als Bilder ausgegeben werden. Die Daten können so in beliebiger Weise weiterverarbeitet und dann gespeichert oder ausgegeben werden.

Komponenten des Bilderzeugungssystems 40 können überwiegend oder vollständig in Form von Softwareelementen auf einem geeigneten Prozessor realisiert sein. Insbesondere können auch die Schnittstellen zwischen einzelnen Komponenten, wie zum Beispiel zwischen Komponenten 25a, 25b der Bilderzeugungseinheit 25 rein softwaremäßig ausgebildet sein. Erforderlich ist lediglich, dass Zugriffsmöglichkeiten auf geeignete Speicherbereiche bestehen, in denen die Daten geeignet zwischengelagert und jederzeit wieder aufgerufen und aktualisiert werden können.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

**Patentansprüche**

1. Verfahren zum Erzeugen von CT-Bilddaten (BD) mit spektraler Information von einem Untersuchungsbereich eines Patienten (P), aufweisend die Schritte:

- Bestrahlen des Untersuchungsbereichs mit polychromatischer Röntgenstrahlung,
- Erfassen von spektral aufgelösten Projektionsmessdaten ($D^1_i$, $D^2_i$) von dem Untersuchungsbereich mit Hilfe eines photonenzählenden Detektors (16) mit mehreren Energieschwellen, wobei die spektral aufgelösten Projektionsmessdaten ($D^1_i$, $D^2_i$) einen ersten Projektionsmessdatensatz ($D^1_i$), welcher einer ersten Energieschwelle zugeordnet ist, und mindestens einen zweiten Projektionsmessdatensatz ($D^2_i$), welcher mindestens einer zweiten Energieschwelle zugeordnet ist, umfassen,
- Erzeugen mindestens eines zweiten Projektionsmessdatensatzes ($M^2_j$) mit reduzierter Auflösung auf Basis des mindestens einen zweiten Projektionsmessdatensatzes ($D^2_i$),
- Übermitteln des ersten Projektionsmessdatensatzes ($D^1_i$) und des mindestens einen zweiten Projektionsmessdatensatzes ($M^2_j$) mit reduzierter Auflösung an eine Bilderzeugungseinheit (25),
- Übertragen der höheren Auflösung des ersten Projektionsmessdatensatzes ($D^1_i$) auf den mindestens einen zweiten Projektionsmessdatensatz ($M^2_j$) mit reduzierter Auflösung und/oder auf einen auf dem mindestens einen zweiten Projektionsmessdatensatz ($M^2_j$) mit reduzierter Auflösung basierenden Bilddatensatz (BD2).

2. Verfahren nach Anspruch 1, wobei ein Kombinationsbild (BD) mit den folgenden Schritten erzeugt wird:

- Rekonstruieren eines ersten Bilddatensatzes (BD1) und mindestens eines zweiten Bilddatensatzes (BD2) auf Basis des ersten Projektionsmessdatensatzes ($D^1_i$) und auf Basis des mindestens einen zweiten Projektionsmessdatensatzes ($M^2_j$) mit reduzierter Auflösung ($M^2_j$) unter Anwendung der Übertragung der Auflösung des ersten Projektionsmessdatensatzes ($D^1_i$) auf den mindestens einen zweiten Projektionsmessdatensatz ($M^2_j$) mit reduzierter Auflösung und/oder auf den mindestens einen zweiten Bilddatensatz (BD2),
- Erzeugen eines Kombinationsbildes (BD) durch Kombinieren des ersten Bilddatensatzes (BD1) und des mindestens einen zweiten Bilddatensatzes (BD2).

3. Verfahren nach Anspruch 1 oder 2, wobei die erste Energieschwelle einen niedrigeren Energiewert aufweist als die mindestens eine zweite Energieschwelle.

4. Verfahren nach einem der vorstehenden Ansprüche, wobei die reduzierte Auflösung des mindestens einen zweiten Projektionsmessdatensatzes ($M^2_j$) mit reduzierter Auflösung durch eine Reduktion der Auflösung in mindestens einer der folgenden Richtungen des Detektors (16) erzeugt wird:

- in Kanalrichtung,
- in Zeilenrichtung,
- in Projektionsrichtung.

5. Verfahren nach Anspruch 4, wobei die reduzierte Auflösung des mindestens einen zweiten Projektionsmessdatensatzes ($M^2_j$) durch Reduktion der Auflösung in Kanalrichtung, Zeilenrichtung und Projektionsrichtung erfolgt.

6. Verfahren nach einem der vorstehenden Ansprüche, wobei der erste Projektionsmessdatensatz ($D^1_i$) mit einer kürzeren Frame-Zeit akquiriert wird als der mindestens eine zweite Projektionsmessdatensatz ($D^2_i$).

7. Verfahren nach einem der vorstehenden Ansprüche, wobei das Übertragen der höheren Auflösung mit Hilfe eines Bandfilters erfolgt.

8. Verfahren nach Anspruch 7, wobei eine Regularisierung der Übertragung der höheren Auflösung durch Gradientenvergleich erfolgt.

9. Verfahren nach einem der vorstehenden Ansprüche, wobei der Transfer der höheren Auflösung im Rahmen der Erzeugung der Bilddaten (BD) in einem der folgenden Prozessstadien erfolgt:

- vor der Vorverarbeitung der Projektionsmessdaten (D1i, D2i),
- vor der Rückprojektion der Projektionsmessdaten (D1i, D2i),
- nach der Rückprojektion der Projektionsmessdaten (D1i, D2i),
- nach einer Materialzerlegung der Projektionsmessdaten ($D^1_i$, $D^2_i$) oder Bilddaten (BD).

10. Verfahren nach einem der vorstehenden Ansprüche, wobei zum Übertragen der Auflösung des ersten Projektions-

messdatensatzes ($D^1_i$) auf den mindestens einen zweiten Projektionsmessdatensatz ($M^2_j$) mit reduzierter Auflösung und/oder auf einen auf dem mindestens einen zweiten Projektionsmessdatensatz ($M^2_j$) mit reduzierter Auflösung basierenden Bilddatensatz (BD) die folgenden Schritte durchgeführt werden:

- Ermitteln eines ersten Projektionsmessdatensatzes ($M^1_j$) mit reduzierter Auflösung auf Basis des ersten Projektionsmessdatensatzes ($D^1_i$),
- Ermitteln von höher aufgelösten Differenzdaten auf Basis einer Subtraktion des ersten Projektionsmessdatensatzes (D1i) und des ersten Projektionsmessdatensatzes (M1j) mit reduzierter Auflösung,
- Addieren der höher aufgelösten Differenzdaten zu dem zweiten Projektionsmessdatensatz ($T^2_i$, $M^2_j$) mit reduzierter Auflösung.

11. Bilderzeugungssystem (40), aufweisend:

- einen quantenzählenden Detektor (16) mit mehreren Energieschwellen zum Erfassen von spektral aufgelösten Projektionsmessdaten ($D^1_i$, $D^2_i$) von einem Untersuchungsbereich eines Patienten (P), wobei die spektral aufgelösten Projektionsmessdaten ($D^1_i$, $D^2_i$) einen ersten Projektionsmessdatensatz ($D^1_i$), welcher einer ersten Energieschwelle zugeordnet ist, und mindestens einen zweiten Projektionsmessdatensatz ($D^2_i$), welcher mindestens einer zweiten Energieschwelle zugeordnet ist, umfassen,
- eine Datenreduktionseinheit (16a) zum Erzeugen mindestens eines zweiten Projektionsmessdatensatzes ($M^2_j$) mit reduzierter Auflösung auf Basis des mindestens einen zweiten Projektionsmessdatensatzes ($D^2_i$),
- eine Datenübermittlungseinheit (17) zum Übermitteln der Projektionsmessdaten (D1i, M2j) von dem quantenzählenden Detektor (16) an eine Bilderzeugungseinheit (25),
- die Bilderzeugungseinheit (25) zum Übertragen der Auflösung des ersten Projektionsmessdatensatzes ($D^1_i$) auf den mindestens einen zweiten Projektionsmessdatensatz ($M^2_j$) mit reduzierter Auflösung und/oder auf einen auf dem mindestens einen zweiten Projektionsmessdatensatz ($M^2_j$) mit reduzierter Auflösung basierenden Bilddatensatz (BD2) und zum Rekonstruieren von Bilddaten (BD) auf Basis der übermittelten Projektionsmessdaten ($D^1_i$, $M^2_j$).

12. Computertomographiesystem (1), aufweisend:

- eine Röntgenquelle (15) zum Bestrahlen eines Untersuchungsbereichs eines Patienten (P) mit polychromatischer Röntgenstrahlung,
- ein Bilderzeugungssystem (40) nach Anspruch 11.

13. Computerprogrammprodukt mit einem Computerprogramm, welches direkt in eine Speichereinrichtung eines Computertomographiesystems (1) ladbar ist, mit Programmabschnitten, um alle Schritte eines Verfahrens nach einem der Ansprüche 1 bis 10 auszuführen, wenn das Computerprogramm in dem Computertomographiesystem (1) ausgeführt wird.

14. Computerlesbares Medium, auf welchem von einer Rechnereinheit einlesbare und ausführbare Programmabschnitte gespeichert sind, um alle Schritte eines Verfahrens nach einem der Ansprüche 1 bis 10 auszuführen, wenn die Programmabschnitte von der Rechnereinheit ausgeführt werden.

**Claims**

1. Method for generating CT image data (BD) with spectral information from an examination region of a patient (P), comprising the steps:

- exposing the examination region to polychromatic X-radiation,
- capturing spectrally resolved projection measurement data ($D^1_i$, $D^2_i$) from the examination region with the aid of a photon counting detector (16) having a plurality of energy thresholds, wherein the spectrally resolved projection measurement data ($D^1_i$, $D^2_i$) comprises a first projection measurement data record ($D^1_i$), which is assigned to a first energy threshold, and at least one second projection measurement data record ($D^2_i$), which is assigned to at least one second energy threshold,
- generating at least one second projection measurement data record ($M^2_j$) with reduced resolution on the basis of the at least one second projection measurement data record ($D^2_i$),
- transmitting the first projection measurement data record ($D^1_i$) and the at least one second projection meas-

urement data record ($M^2_j$) with reduced resolution to an image generation unit (25),
- transferring the higher resolution of the first projection measurement data record ($D^1_i$) onto the at least one second projection measurement data record ($M^2_j$) with reduced resolution and/or onto an image data record (BD2) which is based on the at least one second projection measurement data record ($M^2_j$) with reduced resolution.

2. Method according to claim 1, wherein a combination image (BD) is generated by means of the following steps:

- reconstructing a first image data record (BD1) and at least one second image data record (BD2) on the basis of the first projection measurement data record ($D^1_i$) and on the basis of the at least one second projection measurement data record ($M^2_j$) with reduced resolution ($M^2_j$), applying the transfer of the resolution of the first projection measurement data record ($D^1_i$) onto the at least one second projection measurement data record ($M^2_j$) with reduced resolution and/or onto the at least one second image data record (BD2),
- generating a combination image (BD) by combining the first image data record (BD1) and the at least one second image data record (BD2).

3. Method according to claim 1 or 2, wherein the first energy threshold has a lower energy value than the at least one second energy threshold.

4. Method according to one of the preceding claims, wherein the reduced resolution of the at least one second projection measurement data record ($M^2_j$) with reduced resolution is generated by means of reducing the resolution in at least one of the following directions of the detector (16):

- in channel direction,
- in line direction,
- in projection direction.

5. Method according to claim 4, wherein the reduced resolution of the at least one second projection measurement data record ($M^2_j$) is achieved by reducing the resolution in channel direction, line direction and projection direction.

6. Method according to one of the preceding claims, wherein the first projection measurement data record ($D^1_i$) is acquired using a shorter frame time than the at least one second projection measurement data record ($D^2_i$).

7. Method according to one of the preceding claims, wherein the transfer of the higher resolution takes place using a band filter.

8. Method according to claim 7, wherein regularisation of the higher resolution is carried out by means of gradient comparison.

9. Method according to one of the preceding claims, wherein the transfer of the higher resolution takes place as part of the generation of the image data (BD) in one of the following process stages:

- before the preprocessing of the projection measurement data ($D^1_i$, $D^2_i$),
- before the back-projection of the projection measurement data ($D^1_i$, $D^2_i$),
- after the back-projection of the projection measurement data ($D^1_i$, $D^2_i$),
- after a material breakdown of the projection measurement data ($D^1_i$, $D^2_i$) or image data (BD).

10. Method according to one of the preceding claims, wherein the following steps are performed for the purpose of transferring the resolution of the first projection measurement data record ($D^1_i$) onto the at least one second projection measurement data record ($M^2_j$) with reduced resolution and/or onto an image data record (BD) which is based on the at least one second projection measurement data record ($M^2_j$) with reduced resolution:

- determining a first projection measurement data record ($M^1_j$) with reduced resolution on the basis of the first projection measurement data record ($D^1_i$),
- determining higher resolution difference data on the basis of a subtraction of the first projection measurement data record ($D^1_i$) and the first projection measurement data record ($M^1_j$) with reduced resolution,
- adding the higher resolution difference data to the second projection measurement data record ($T^2_i$, $M^2_j$) with reduced resolution.

**11.** Image generation system (40) comprising:

- a quantum counting detector (16) having a plurality of energy thresholds for capturing spectrally resolved projection measurement data ($D^1_i$, $D^2_i$) from an examination region of a patient (P), wherein the spectrally resolved projection measurement data ($D^1_i$, $D^2_i$) comprises a first projection measurement data record ($D^1_i$), which is assigned to a first energy threshold, and at least one second projection measurement data record ($D^2_i$), which is assigned to at least one second energy threshold,
- a data reduction unit (16a) for generating at least one second projection measurement data record ($M^2_j$) with reduced resolution on the basis of the at least one second projection measurement data record ($D^2_i$),
- a data transmission unit (17) for transmitting the projection measurement data ($D^1_i$, $M^2_j$) from the quantum counting detector (16) to an image generation unit (25),
- the image generation unit (25) for transferring the resolution of the first projection measurement data record ($D^1_i$) onto the at least one second projection measurement data record ($M^2_j$) with reduced resolution and/or onto an image data record (BD) which is based on the at least one second projection measurement data record ($M^2_j$) with reduced resolution, and for reconstructing image data (BD) on the basis of the transmitted projection measurement data ($D^1_i$, $M^2_j$).

**12.** Computer tomography system (1) comprising:

- an X-ray source (15) for irradiating an examination region of a patient (P) with polychromatic X-radiation,
- an image generation system (40) according to claim 11.

**13.** Computer program product comprising a computer program which can be loaded directly into a memory device of a computer tomography system (1), with program sections for executing all of the steps of a method according to one of claims 1 to 10 when the computer program is executed in the computer tomography system (1).

**14.** Computer-readable medium, on which are stored program steps that can be read in and executed by a computer unit in order to execute all of the steps of a method according to one of claims 1 to 10 when the program sections are executed by the computer unit.

**Revendications**

**1.** Procédé de production de données (BD) d'images CT, comportant des informations spectrales d'une région à étudier d'un patient (P), comportant les stades :

- exposition de la région à étudier à du rayonnement X polychromatique,
- détection de données ($D^1_i$, $D^2_i$) de mesure de projection à résolution spectrale de la région à étudier à l'aide d'un détecteur (16) comptant les photons à plusieurs seuils d'énergie, les données ($D^1_i$, $D^2_i$) de mesure de projection à résolution spectrale comprenant un premier jeu ($D^1_i$) de mesure de projection, qui est associé à un premier seuil d'énergie et au moins un deuxième jeu ($D^2_i$) de mesure de projection, qui est associé à au moins un deuxième seuil d'énergie,
- production d'au moins un deuxième jeu ($M^2_j$) de données de mesure de projection à résolution réduite sur la base du au moins un deuxième jeu ($D^2_i$) de données de mesure de projection,
- transmission du premier jeu ($D^1_i$) de données de mesure de projection et du au moins un deuxième jeu ($M^2_j$) de données de mesure de projection à résolution réduite à une unité (25) de production d'image,
- transfert de la résolution plus grande du premier jeu ($D^1_i$) de données de mesure de projection au au moins un deuxième jeu ($M^2_j$) de données de mesure de projection à résolution réduite et/ou à un jeu (BD2) de données d'image reposant sur le au moins un deuxième jeu ($M^2_j$) de données de mesure de projection à résolution réduite.

**2.** Procédé suivant la revendication 1, dans lequel on produit une image (BD) de combinaison par les stades suivants :

- reconstruction d'un premier jeu (BD1) de données d'image et d'au moins un deuxième jeu (BD2) de données d'image sur la base du premier jeu ($D^1_i$) de données de mesure de projection et sur la base du au moins un deuxième jeu ($M^2_j$) de données de mesure de projection à résolution ($M^2_j$) réduite, en utilisant le transfert de la résolution du premier jeu ($D^1_i$) de données de mesure de projection au au moins un deuxième jeu ($M^2_j$) de données de mesure de projection à résolution réduite et/ou au au moins un deuxième jeu (BD2) de données d'image,

- production d'une image (BD) de combinaison par combinaison du premier jeu (BD1) de données d'image et du au moins un deuxième jeu (BD2) de données d'image.

3. Procédé suivant la revendication 1 ou 2, dans lequel le premier seuil d'énergie a une valeur d'énergie plus petite que le au moins un deuxième seuil d'énergie.

4. Procédé suivant l'une des revendications précédentes, dans lequel on produit la résolution réduite du au moins un deuxième jeu ($M^2_j$) de données de mesure de projection à résolution réduite par une réduction de la résolution dans au moins l'une des directions suivantes du détecteur (16) :

   - dans la direction de canal,
   - dans la direction des lignes,
   - dans la direction de projection.

5. Procédé suivant la revendication 4, dans lequel la résolution réduite du au moins un deuxième jeu ($M^2_j$) de données de mesure de projection s'effectue par réduction de la résolution dans la direction du canal, la direction des lignes et la direction de projection.

6. Procédé suivant l'une des revendications précédentes, dans lequel on acquiert le premier jeu ($D^1_i$) de données de mesure de projection en un temps de trame plus court que le au moins un deuxième jeu ($D^2_i$) de données de mesure de projection.

7. Procédé suivant l'une des revendications précédentes, dans lequel le transfert de la résolution plus grande s'effectue à l'aide d'un filtre passe bande.

8. Procédé suivant la revendication 7, dans lequel une régularisation du transfert de la résolution plus grande s'effectue par une comparaison de gradient.

9. Procédé suivant l'une des revendications précédentes, dans lequel le transfert de la résolution plus grande s'effectue dans le cadre de la production des données (BD) d'image dans l'un des stades de processus suivants :

   - avant le prétraitement des données ($D^1_i$, $D^2_i$) de mesure de projection,
   - avant la rétroprojection des données ($D^1_i$, $D^2_i$) de mesure de projection,
   - après la rétroprojection des données ($D^1_i$, $D^2_i$) de mesure de projection,
   - après une décomposition matérielle des données ($D^1_i$, $D^2_i$) de mesure de projection ou des données (BD) d'image.

10. Procédé suivant l'une des revendications précédentes, dans lequel, pour transférer la résolution du premier jeu ($D^2_i$) de données de mesure de projection au au moins un deuxième jeu ($M^2_j$) de données de mesure de projection à résolution réduite et/ou à un jeu (BD) de données d'image reposant sur le au moins un deuxième jeu ($M^2_j$) de données de mesure de projection à résolution réduite, on effectue les stades suivants :

   - détermination d'un premier jeu ($M^1_j$) de données de mesure de projection à résolution réduite sur la base du premier jeu ($D^1_i$) de données de mesure de projection à résolution réduite sur la base du premier jeu ($D^1_i$) de données de mesure de projection,
   - détermination de données de différence à grande résolution sur la base d'une soustraction du premier jeu ($D^1_i$) de données de mesure de projection et du premier jeu ($M^1_i$) de données de mesure de projection à résolution réduite,
   - addition de données de différence à grande résolution au deuxième jeu ($T^2_i$, $M^2_j$) de données de mesure de projection à résolution réduite.

11. Système (40) de production d'images, comportant :

   - un détecteur (16) de comptage de quanta à plusieurs seuils d'énergie pour détecter des données ($D^1_i$, $D^2_i$) de mesure de projection à résolution spectrale d'une région à étudier d'un patient (P), les données ($D^1_i$, $D^2_i$) de mesure de projection à résolution spectrale comprenant un premier jeu ($D^1_i$) de données de mesure de projection, qui est associé à un premier seuil d'énergie, et au moins un deuxième jeu ($D^2_i$) de données de mesure de projection, qui est associé à au moins un deuxième seuil d'énergie,

- une unité (16a) de réduction de données pour produire au moins un deuxième jeu ($M^2_j$) de données de mesure de projection à résolution réduite sur la base du au moins un deuxième jeu ($D^2_i$) de données de mesure de projection,
- une unité (17) de transmission de données pour transmettre les données ($D1_i$, $M2_j$) de mesure de projection du détecteur (16) de comptage de quanta à une unité (25) de production d'images,
- l'unité (25) de production d'images, pour transférer la résolution du premier jeu ($D^1_i$) de données de mesure de projection au au moins un deuxième jeu ($M^2_j$) de données de mesure de projection à résolution réduite et/ou à un jeu (BD2) de données d'image reposant sur le au moins un deuxième jeu ($M^2_j$) de données de mesure de projection à résolution réduite et pour reconstruire des données (BD) d'image sur la base des données ($D^1_i$, $M^2_j$) de mesure de projection transmises.

12. Système (1) de tomographie à ordinateur, comportant :

- une source (15) de rayons X pour soumettre une région à étudier d'un patient (P) à une exposition à du rayonnement X polychromatique,
- un système (40) de production d'image suivant la revendication 11.

13. Produit de programme d'ordinateur, ayant un programme d'ordinateur, qui peut être chargé directement dans un dispositif de mémoire d'un système (1) de tomographie à ordinateur, comprenant des parties de programme pour effectuer tous les stades d'un procédé suivant l'une des revendications 1 à 10, lorsque le programme d'ordinateur est réalisé dans le système (1) de tomographie à ordinateur.

14. Support déchiffrable par ordinateur, sur lequel sont mémorisées des parties de programme pouvant être lues et réalisées par une unité de calcul pour effectuer tous les stades d'un procédé suivant l'une des revendications 1 à 10, lorsque les parties du programme sont réalisées par l'unité de calcul.

## FIG 1

100

1.I

1.II

$D^2_i$ ⟋ ⟋ $D^1_i$          ⟋ $D^1_i$

1.III          1.VI

$M^2_j$ ⟋ ⟋ $D^1_i$

1.IV  $U(D^1_i, M^2_j)$

$M^2_j$ ⟋          $D^1_i$ ⟋          ⟋ $M^1_j$

1.V

$M^2_j$ ⟋ ⟋ $T^2_i$

1.VII  $F^2_i$

$D^1_i$ ⟋ ⟋ $F^2_i$

1.VIII

BD1 ⟋ ⟋ BD2

1.IX  BD

FIG 2

$D^1i=$  4  6  5  13  12  16

$D^2i=$  1  5  3  11  9  11

$D^1i=$  4  6  5  13  12  16

$M^2j=$  3  7  10

FIG 3

$D^1i=$  4  6  5  13  12  16

$T^2i=$  3  3  7  7  10  10

$M^1j=$  5  9  14

$F^2i=$  2.4  3.8  8.6
3.6  10.1  11.4

FIG 4

FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **PICHA SHUNHAVANICH ; NORBERT J.PELC.** Lossy Compression of Projection Data from Photon Counting Detectors. *4TH INTERNATIONAL CONFERENCE ON IMAGE FORMATION IN X-RAY COMPUTED TOMOGRAPHY,* 18. Juli 2016, 467-470